# FASCICULE DE BREVET EUROPEEN

(11) **EP 2 150 812 B1**
(45) Date de publication et mention de la délivrance du brevet: **01.04.2020**
(21) Numéro de dépôt: 08709771.3
(22) Date de dépôt: 06.02.2008
(51) Int. Cl.: G01N 33/08, G01N 21/85, A01K 43/00

(54) **PROCEDE ET INSTALLATION DE MIRAGE DES OEUFS AVEC DETECTION DE LA PRESENCE D'UNE CHAMBRE A AIR**
EIERDURCHLEUCHTUNGSEINRICHTUNG UND VERFAHREN MIT LUFTZELLENNACHWEIS
EGG CANDLING FACILITY AND METHOD WITH AIR CELL DETECTION

(30) Priorité: 06.02.2007 FR 0700840
(43) Date de publication de la demande: 10.02.2010
(73) Titulaire: Visio Nerf, 49340 Nuaille (FR)
(72) Inventeur: ROBERT, Pierre, 49300 Cholet (FR); SOMVILLE, Olivier, 49300 Cholet (FR)
(74) Mandataire: Fédit-Loriot
(86) Numéro de dépôt international: PCT/IB2008/000259
(87) Numéro de publication internationale: WO 2008/096236

(56) Documents cités:
- WO-A-2004/023136
- WO-A2-02/086495
- US-A1- 2005 122 524
- US-A1- 2006 038 978
- DATABASE EPODOC EUROPEAN PATENT OFFICE, THE HAGUE, NL; BG-9963295-A, BG61856-B1 31 juillet 1998 (1998-07-31), KRIVOSHIEV GEORGI, P. ET AL: "METHOD AND DEVICE FOR AUTOMATIC CLASSIFICATION OF EGGS ACCORDING TO THEIR FRESHNESS" XP002478018 -& BG 61 856 B1 (KRIVOSHIEV GEORGI P [BG]; CHALAKOVA DIMITROVA [BG]; FILIPOV STEFAN B [) 31 juillet 1998 (1998-07-31)

## Description

La présente invention concerne les techniques industrielles d'examen d'oeufs de volailles qu'il est convenu d'englober dans la notion de mirage des oeufs. Les techniques de mirage des oeufs sont couramment appliquées en aviculture, où elles servent notamment, par un examen visiométrique des oeufs, à déterminer un état caractéristique de chacun, notamment d'ordre physiologique, en fonction de critères prédéfinis.

Ne serait-ce que par les brevets déposés précédemment par la société déposante, on connaît ainsi des installations de mirage des oeufs dans lesquelles les oeufs, disposés en rangées et colonnes dans les alvéoles de casiers, sont entraînés à défiler à travers un poste dit de visiométrie, où chaque oeuf est amené à intercepter un faisceau lumineux dont on mesure l'atténuation à la traversée de l'oeuf. Une telle installation est décrite par exemple dans le brevet français FR 27 685 17, la demande internationale de brevet WO 99/14589 et le brevet français FR 28 957 98. Les oeufs y sont examinés rangée par rangée au moyen d'une série de diodes électro-luminescentes qui les illuminent par en dessous et d'une série de capteurs associés disposés au-dessus d'eux, avantageusement du type à couplage de charge et intégrés dans une caméra vidéo. Les images ainsi saisies sont traitées par des moyens informatiques programmés pour mettre en oeuvre des modules logiciels qui réalisent automatiquement une comparaison entre le niveau lumineux du faisceau émergeant de chaque oeuf et des valeurs seuil prédéterminées.

En général, ces valeurs seuil sont définies par des essais préalables portant sur des lots échantillons examinés dans les mêmes conditions d'illumination et de saisie d'images, encore que l'on peut aussi comparer les atténuations lumineuses d'un oeuf à l'autre- pour décider d'un tri entre les oeufs à atténuation relativement forte et les oeufs à atténuation relativement faible.

En application particulièrement fréquente des techniques de ce genre, on trouve la détermination de l'état de fécondation des oeufs. Suivant le niveau d'opacité dont ils témoignent, on distingue les oeufs fécondés des oeufs non fécondés, couramment appelés oeufs clairs. Un tel examen est à effectuer notamment en début d'une procédure d'incubation des oeufs (sur des oeufs fraîchement pondus ou plus couramment sur des oeufs ayant été conservés au froid entre-temps), pour apporter un moyen rapide de trier les oeufs et recueillir sélectivement les oeufs convenablement fécondés en excluant les oeufs clairs. Seuls les premiers sont destinés soit, le plus souvent, à être dirigés vers les incubateurs et éclosoirs où naîtront les poussins, soit à servir comme milieu de culture de souches de micro-organismes, en particulier dans la fabrication de vaccins. US 2006/038978 A1 divulgue un procédé de mirage des œufs par visiométrie pour produire une image de chaque œuf d'une pluralité œufs et d'évaluation de la qualité des œufs. Le système d'imagerie est conçu pour imager les veines, les poches d'air et l'emplacement de l'embryon de chaque œuf et comprend un contrôleur pour comparer les veines, les poches d'air et l'embryon à une référence.

Cependant, telle qu'elle est décrite et mise en oeuvre actuellement, la technique a ses limites. Elle manque souvent de sensibilité, en ne permettant pas de discerner différents états parmi les oeufs auxquels l'attribut d'oeuf fécondé est assigné par opposition aux oeufs clairs. Egalement, si la méthode donne correctement satisfaction dans le traitement des oeufs de poule, il n'en est pas de même pour d'autres races animales. On a, en particulier, observé un taux d'erreur non négligeable dans le cas des oeufs de dinde.

L'invention propose de remédier à l'insuffisance des techniques classiques en s'affranchissant des perturbations liées à la présence de la coquille extérieure des oeufs, laquelle est souvent variable en épaisseur et en transparence non seulement d'un lot de ponte à l'autre ou d'une race de volaille à une autre, mais même d'un oeuf à l'autre dans un même lot.

Elle a pour objet un procédé de mirage des oeufs consistant essentiellement à examiner les oeufs par visiométrie dans des conditions d'illumination et de saisie d'image qui sont sensibles à un contraste lumineux s'observant, au sein de chaque oeuf, entre la zone liquide de l'oeuf, celle où croît l'embryon résultant d'une fécondation, et la zone remplie d'air complémentaire qui est éventuellement également présente à l'intérieur de l'oeuf et que l'on appelle chambre à air ("air sac" ou "air bag" en anglais).

On comprend donc qu'alors que dans les mesures classiques, la différence d'atténuation entre oeuf clair et oeuf fécondé s'exprime d'un oeuf à l'autre statistiquement, l'invention exploite la présence de la chambre d'air qui existe normalement dans chaque oeuf examiné pour définir l'état de l'oeuf, non plus en fonction de l'opacité ou la transparence de l'oeuf dans son ensemble, mais en fonction des effets respectifs du liquide et de l'air sur une lumière pénétrant à l'intérieur de l'oeuf. Suivant des modes de mise en oeuvre préférés de l'invention, on opère dans des conditions paramétrées pour être sensibles au contraste lumineux entre zone d'air et zone de liquide au sein de chaque oeuf tel qu'il peut s'observer, sous illumination par un faisceau de lumière focalisé sur l'œuf, sur une image de la lumière rétrodiffusée à partir de chaque oeuf, à l'exclusion de la lumière renvoyée par réflexion sur sous un faisceau focalisé au-delà de sa coquille.

On s'affranchit ainsi du rôle dominant que joue la coquille quand on observe l'ensemble de l'oeuf. Déjà de ce fait, l'invention permet d'élargir notablement le champ d'application du mirage des oeufs, tant en améliorant la sensibilité et la fiabilité des résultats qu'en généralisant les types d'oeufs qui peuvent être traités avec efficacité. Ces avantages trouveront intérêt, par exemple, dans une meilleure évaluation de la fertilité des oeufs et dans un traitement efficace des oeufs de n'importe quelle race de volaille quand il s'agit de répartir les oeufs en fonction de leur état de fécondation pour les orienter vers des destinations différentes. Le tri entre les oeufs clairs et les oeufs fécondés, éventuellement entre les oeufs à embryon mort et les oeufs à embryon vivant, est là l'exemple le plus courant d'une application fort utile pour les installations de production d'oeufs fertiles par insémination artificielle, compte tenu du taux élevé d'échecs de fécondation.

La mise en oeuvre de l'invention entraîne d'autres conséquences bénéfiques, qui sont liées elles au fait qu'elle rend possible de localiser la chambre à air par rapport à la zone liquide à l'intérieur de chaque oeuf. Cette capacité répond notamment aux besoins des utilisations des oeufs comme milieu de culture dans la production de vaccins, quand on souhaite inoculer la souche de vaccin à l'intérieur de la chambre à air sans atteindre l'embryon autrement que par diffusion ultérieure à travers la membrane qui sépare l'un de l'autre, ou même quand on cherche seulement à s'assurer que l'aiguille d'inoculation perce la coquille de l'oeuf au niveau de la zone d'air plutôt qu'au niveau de la zone de liquide, ce qui est préférable pour limiter les risques de pollution ou contamination.

Dans des modes de mise en oeuvre préférés de l'invention, on est donc amené à tirer profit à la fois des avantages en termes de sensibilité et de fiabilité et de la possibilité de localiser la chambre à air de chaque oeuf. On pense ici notamment aux applications en suivi de lots d'oeufs en cours d'incubation à un stade précoce de la croissance des embryons dans les chaînes de production de poussins et à la collecte des oeufs embryonnés pour l'inoculation par une dose de vaccin ou autre souche de culture.

L'invention est en effet tout particulièrement utile pour réaliser un mirage des oeufs à un stade précoce de l'incubation. On profite pleinement de la grande sensibilité des mesures visiométriques à partir du moment où la chambre à air est apparue dans l'oeuf et jusqu'à ce que l'embryon parvienne à un stade de croissance où il remplit quasiment la coquille et où son incidence sur l'opacité de l'ensemble devient prépondérante. C'est pendant cette période qu'il est souhaitable de voir réaliser des examens dits de mirage précoce, pour écarter les oeufs clairs du circuit des oeufs fécondés pour lesquels l'incubation est terminée quand ils sont destinés à des applications en pharmacie, notamment pour la production de vaccins.

Au cours du même examen ou au cours d'un examen similaire, on peut aussi, conformément à une variante avantageuse de mise en oeuvre de l'invention, traiter les signaux d'image obtenus pour déterminer où se situe le point central de la zone couverte dans le plan d'examen par la chambre à air dans chaque oeuf et utiliser le résultat du calcul automatique comme repère caractéristique de la position du sommet de la chambre à air. Il s'agit là d'une application de l'invention impliquant une localisation de la chambre à air dans chaque oeuf qui est plus élaborée que celle qui consiste, de manière plus élémentaire, à déterminer si la chambre à air est présente ou non dans la partie de l'oeuf soumise à l'examen, notamment pour fournir l'information obtenue à un dispositif de traitement automatique des lots d'oeufs examinés.

Dans les modes préférés d'application pratique de l'invention, l'illumination et la saisie d'image s'effectuent suivant le même axe, généralement par le dessus des oeufs. Le matériel utilisé implique alors un masque qui cache aux détecteurs de la caméra de saisie d'image les faisceaux directement réléchis par les coquilles, de manière à ne retenir que la lumière revenant après diffusion à l'intérieur de chaque oeuf, au-delà de sa coquille. Par ailleurs, un faisceau de lumière éclairant les oeufs correctement focalisé est obtenu au mieux en lumière laser. Il peut être produit sous la forme d'un trait de lumière éclairant une rangée d'oeufs observés simultanément. La lumière laser est avantageusement choisie dans la gamme du rayonnement infrarouge.

Ayant fait ressortir dans ce qui précède le double intérêt de la technique suivant l'invention pour les applications dans le domaine du mirage précoce des oeufs, on s'intéressera maintenant à mieux décrire l'objet de l'invention sous d'autres aspects, concernant ses modes de mises en oeuvre préférés au niveau du procédé et de l'installation de mirage des oeufs proprement dite.

Leur description s'appuie sur les illustrations des figures qu'elle comporte, parmi lesquelles :
- La figure 1 se réfère à un oeuf d'une rangée déterminée passant dans la zone de vision ;
- La figure 2 montre schématiquement comment se présente l'image saisie par la caméra à ce moment ;
- Les figures comparatives 3a et 3b illustrent l'intérêt que l'on a à assurer l'éclairage en illumination de chaque oeuf suivant un trait de lumière plutôt que d'utiliser un éclairage ponctuel ;

- La figure 4 illustre le fonctionnement du dispositif en cours d'examen d'une rangée d'un casier d'oeufs en trois étapes de saisie d'image successives, faisant l'objet respectivement des figures 4a, 4b ,4c ;
- Et la figure 5 illustre comment sont constituées les trois images correspondantes pour trois oeufs adjacents de la même rangée qui sont supposés différer l'un de l'autre par la disposition de la chambre à air par rapport à l'axe d'illumination et saisie d'image.

Dans les conditions préférées de mise en oeuvre de l'invention, telle qu'elle s'applique notamment dans le domaine de la production d'oeufs et de l'élevage industriel de volailles, les opérations de mirage s'effectuent en faisant défiler les casiers d'oeufs l'un après l'autre à travers le poste de visiométrie ainsi qu'il est décrit plus en détail dans les textes de brevets déjà cités.

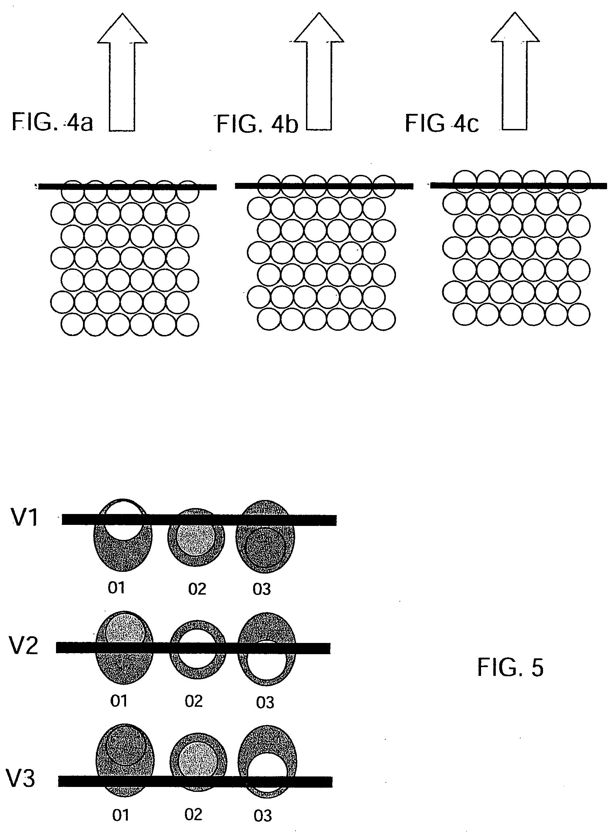

Les figures 4 montrent la disposition des oeufs dans leurs alvéoles respectives en vue de dessus. Dans le cas représenté, la disposition respecte une répartition en réseau à mailles hexagonales, mais cette disposition n'est pas limitative. On se référera avec utilité à la description figurant dans le brevet français FR 270685017, qui décrit des modes de fonctionnement d'un poste de visiométrie dans des conditions spécifiquement adaptées à la disposition en mailles hexagonales.

Par contre, là où ce brevet antérieur de la demanderesse décrivait un examen des oeufs sous un éclairage par le dessous à travers des lumières ménagées dans le casier dans l'axe de chaque alvéole, l'éclairage s'effectue dans le cas présent par des diodes émettrices situées, comme la caméra de prise de vues, au-dessus du trajet des casiers d'oeufs.

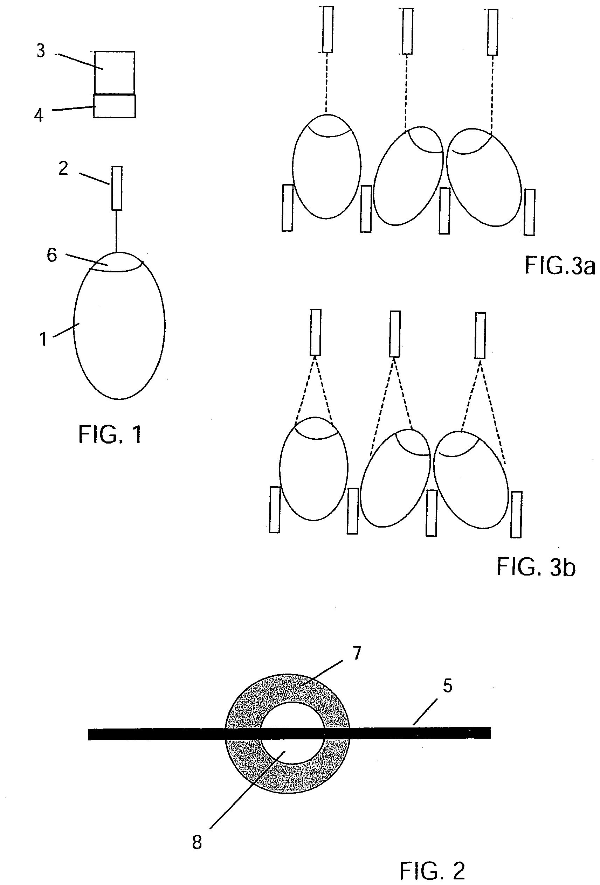

C'est ainsi que l'on a schématiquement représenté sur la figure 1, pour un oeuf 1, d'une part une diode 2 émettrice d'un faisceau laser l'illuminant, supposé ici en lumière infrarouge, d'autre part une caméra 3 avec son système optique d'entrée 4, pour ce qui concerne ceux de ses éléments qui sont situés fonctionnellement en regard de l'oeuf 1, de telle sorte qu'ils sont sensibles à la lumière rétrodiffusée par cet oeuf. Le système optique de la caméra est réglé pour une bonne qualité de l'image saisie à ce niveau. Elle est située sensiblement dans le même axe que le faisceau d'illumination. Il lui est alors associé des moyens appropriés pour lui cacher la lumière directement réfléchie par l'oeuf, qui lui parviendrait en retour sans avoir pénétré à l'intérieur l'oeuf. Ces moyens impliquent un masque dont on reparlera plus loin.

On a aussi illustré sur la figure 1 que, dans sa position normale, l'oeuf 1 présente une chambre à air 6 dans sa partie supérieure, au sommet ou au voisinage du sommet du grand diamètre de sa forme ovoïde. Conformément à l'invention, le faisceau de lumière émis depuis la diode émettrice 2, est focalisé à l'intérieur de cette chambre à air, au-delà de la coquille qui enveloppe l'oeuf.

En pratique, pour une installation industrielle de mirage des oeufs, les oeufs sont ainsi examinés rangée par rangée, en considérant chaque rangée dans la direction transversale à la direction de déplacement des casiers. Quant à l'illumination des oeufs, si elle peut être réalisée de manière ponctuelle pour chaque oeuf, on a préféré dans le cas présent un éclairage linéaire projetant un trait de lumière en travers de chaque oeuf examiné. Dans le cas particulier choisi pour illustrer au mieux l'invention, cela se traduit par une barre d'éclairage rectiligne courant transversalement à la direction de défilement des casiers d'oeufs dans le poste de visiométrie, et s'étendant même, sous une forme de réalisation particulièrement commode, à travers toute la rangée à laquelle chaque oeuf appartient, comme il est apparaît suivant un trait noir 5 sur la figure 2.

La même figure 2 illustre l'image lumineuse restituée par la caméra au niveau de l'oeuf 1 en mode monochrome. On observe la présence d'un anneau grisé 7, autour d'une partie centrale 8 relativement blanche. De fait, la zone liquide de l'oeuf entourant la chambre à air dans le plan de focalisation du faisceau d'illumination ressort en sombre, compte tenu de son caractère semi-opaque, autour d'une zone claire, correspondant à la chambre à air, nettement plus transparente.

Le système de traitement des images numériques associé à la caméra de prise de vues calcule, à partir de l'image saisie pour chaque oeuf, le niveau de contraste entre chambre à air et partie liquide, et par comparaison avec des valeurs prédéterminées, il détermine si ce contraste est significatif ou non de la présence d'un embryon.

Par comparaison avec des seuils appropriés ou par comparaison statistique des images obtenues d'un oeuf à l'autre, le système informatique de traitement des images numériques peut également déterminer, en dehors de la gamme correspondant à un contraste significatif de la présence correcte d'une chambre à air, d'une part si l'oeuf n'a pas été fécondé, auquel cas il apparaît relativement clair dans sa totalité, d'autre part si l'absence de chambre à air dans une partie liquide relativement sombre traduit le fait que l'oeuf soit disposé tête en bas.

Dans le premier cas, les oeufs clairs sont écartés du circuit de l'incubation pour être éventuellement récupérés vers une autre destination. Dans le second cas, les oeufs peuvent être recyclés à l'opération de mirage après avoir été retournés dans une alvéole réceptrice pour présenter leur chambre à air sur le dessus et non plus en dessous.

Les figures 3a et 3b illustrent l'intérêt que l'on a à réaliser l'éclairage suivant un plan projetant un trait de lumière plutôt que par un faisceau ponctuel.

Un faisceau ponctuel risque de se trouver focalisé à côté de la chambre à air si l'oeuf ne présente pas sa chambre à air assez précisément dans l'axe du faisceau. Un éclairage par une barre transversale à la direction de déplacement permet au système de visiométrie d'être néanmoins sensible au contraste entre chambre à air et zone liquide du fait que la lumière focalisée atteint l'intérieur de l'oeuf à travers sa coquille sur une partie au moins de sa largeur.

C'est ainsi que pour des oeufs inclinés vers la droite ou vers la gauche par rapport à la verticale dans leurs alvéoles respectives, les faisceaux ponctuels correspondants, en lumière projetée verticalement, atteignent l'oeuf à côté de la chambre à air sur la figure 3a, alors que pour des oeufs pareillement inclinés sur la figure 3b, les faisceaux s'évasant de part et d'autre de la verticale permettent la détection de la chambre à air en atteignant celle-ci latéralement suivant un rayon.

Réaliser l'illumination au moyen d'un faisceau finement focalisé pénétrant au-delà de la coquille de l'oeuf, au niveau de la chambre à air, fonctionne de pair avec le fait d'effectuer la saisie d'images par détection de la lumière qui est rétrodiffusée à travers la coquille, pour avoir été principalement réfléchie à l'interface entre zone d'air et zone de liquide (au niveau de la fine membrane qui les sépare l'une de l'autre), puis éventuellement atténuée en traversant de la zone liquide entourant la chambre à air (sur une épaisseur relativement faible par rapport à celle qu'il faudrait traverser dans une mesure d'atténuation à travers l'oeuf dans son ensemble).

L'ensemble conduit à des images de contraste sur lesquelles on observe le degré de contraste lumineux entre zone liquide et zone d'air par des outils de traitement automatique d'images numériques pilotés par des programmes informatiques pré-enregistrés. L'atténuation à la traversée de la coquille est sans incidence sur la comparaison des luminosités relevées pour chaque oeuf, si bien que l'on s'affranchit ainsi de l'influence d'une coquille plus ou moins épaisse dans le résultat des mesures.

Du fait que l'on s'intéresse à détecter par caméra la lumière renvoyée par l'oeuf depuis l'intérieur de celui-ci, on a intérêt à éviter que la caméra pâtisse d'un phénomène d'éblouissement par de la lumière réfléchie vers elle par la coquille. A cette fin, l'invention prévoit notamment de disposer un masque devant la caméra sur le trajet du faisceau directement réfléchi par la coquille des oeufs à partir du trait de lumière dirigé vers eux. C'est ce masque qui se traduit sur l'image obtenue par le trait noir 5 que l'on a fait ressortir sur la figure 2.

Pour des questions de commodité de réalisation technologique, ce masque est continu sur toute la largeur des rangées d'oeufs en défilement longitudinal, alors même que l'on a supposé plus haut que l'on utilise des sources individuelles, affectées chacune à l'un des oeufs de chaque rangée en cours d'examen, ce qui permet, en variante, d'organiser des commandes différentielles des conditions d'illumination des différents oeufs, en intensité ou en durée.

En réalisant les opérations de visiométrie sous illumination suivant un trait transversal à la direction de déplacement, on résout le problème que peuvent poser des oeufs inclinés latéralement de la manière qui a été expliquée plus haut, en référence aux figures 3a et 3b.

Pour traiter pleinement le cas d'oeufs pouvant être inclinés aussi dans la direction longitudinale, on peut mettre en oeuvre avec profit le mode de réalisation de l'invention illustré par les figures 4 et 5. Au niveau de chaque rangée d'oeufs, on effectue trois opérations successives d'illumination et saisie d'images pendant que chaque rangée d'oeufs se déplace sous l'équipement de visiométrie. La figure 4 montre comment se place la barre de sources de lumière dans chacune des opérations de saisie d'images respectivement. La figure 5 illustre les images obtenues pour trois oeufs adjacents orientés différemment.

Sur la première des images V1 au cours du défilement, la chambre de l'oeuf 01, qui est orienté vers l'avant, est bien visible, alors qu'elle l'est moins dans l'image intermédiaire V2 et qu'elle ne l'est plus guère dans la dernière image V3. A l'inverse, la chambre à air de l'oeuf 03, supposé incliné vers l'arrière, est invisible sur la première image V1, peu éclairée sur l'image intermédiaire V2, et bien éclairée sur la dernière image V3. Pour l'oeuf 02, supposé bien vertical, c'est l'image intermédiaire V2 qui est la plus nette à faire ressortir le contraste entre chambre à air et partie liquide embryonnée.

Le traitement d'images exploitant les images numériques saisies conformément à ces figures est programmé pour mémoriser les données des trois images successivement saisies pour chaque rangée au cours du défilement des oeufs sous la caméra et pour sélectionner pour chaque oeuf celle des trois images dans laquelle le contraste est le plus fort entre la zone d'air de l'oeuf et la zone de liquide. L'information correspondante est utilisée comme témoin de la présence de la chambre à air, ainsi éventuellement que de sa position.

L'ensemble des informations de mirage recueillies sont disponibles pour être exploitées de toute manière connue. Le système informatique associé au poste de visiométrie est avantageusement programmé pour fournir les résultats de l'analyse sous forme d'un signal véhiculant, pour chaque oeuf, une information de coordonnées indicatrice de sa position dans le casier le contenant (notamment par numéro d'ordre dans son rang et numéro d'ordre dans sa colonne) et une information d'accessibilité de chambre à air, indiquant si la présence de la chambre à air a été ou non détectée. De préférence, l'information d'accessibilité est aussi indicatrice de l'orientation du sommet de la chambre à air, par rapport à l'axe d'illumination ou de saisie des images.

Ce signal peut être utilisé en ligne ou en différé pour un traitement ultérieur des oeufs. Il peut aussi servir à commander un système de marquage des oeufs par projection d'encre en fonction de leur état relevé au poste de visiométrie, ou à commander de manière similaire un système d'injection de souches de vaccin par des aiguilles d'inoculation pénétrant à l'intérieur de chaque oeuf en traversant la coquille au niveau de la chambre à air.

Le cas échéant, le signal de commande d'un tel système peut véhiculer une information de position de la chambre à air par rapport à la référence verticale de l'axe d'examen visiométrique dont la présence est exploitable pour commander automatiquement un décalage ponctuel de l'aiguille d'inoculation de manière qu'elle se présente au droit du sommet de la chambre à air. Ce dernier se calcule au plus simple comme se situant au milieu entre les deux points extrêmes de détection de la présence de la chambre à air dans le sens transversal à la direction de déplacement. Son calcul peut aussi prendre en compte un décalage de position dans le sens longitudinal à partir des informations recueillies au cours de l'examen d'un même oeuf suivant des lignes d'éclairage successives.

En variante de mise en oeuvre de l'invention, l'information ainsi obtenue, à un stade précoce du processus de croissance au cours de l'incubation, peut être utilisée pour déterminer l'opportunité de vacciner l'embryon correspondant en injectant une dose de vaccin dans l'oeuf embryonné. On pourra programmer cette injection au choix soit au niveau de la chambre à air, soit au niveau de l'embryon lui-même à un stade approprié de sa croissance, en opérant soit à l'occasion du mirage précoce, soit plutôt au terme de l'incubation avant passage dans l'éclosoir.

Au cours de la description détaillée qui précède, en expliquant comment se déroule le procédé suivant l'invention dans des modes pratiques de réalisation (dont on rappellera toutefois le caractère non limitatif), on aura aussi montré comment est constituée une installation de mirage des oeufs comportant, conformément à l'invention, des moyens propres à la mise en oeuvre des différentes étapes de ce procédé.

Dans le cadre d'une installation de mirage des oeufs dans laquelle les oeufs sont entraînés à défiler en rangées successives, on a décrit en particulier comment sont constitués les moyens d'illumination et la caméra de saisie d'images numériques du poste de visiométrie, ainsi que les moyens de traitement automatique des signaux d'images qui exploitent ces signaux pour déterminer si une chambre à air est présente dans le champ de l'examen à l'intérieur de chaque oeuf, et le cas échéant pour déterminer sa localisation par rapport à l'axe d'observation.

On aura aussi fait ressortir deux moyens d'observer le contraste entre zone d'air et zone de liquide dans chaque oeuf pour plusieurs points distincts en travers de chaque oeuf. L'un procède par éclairage des oeufs dans chaque rangée en cours d'examen par un faisceau d'illumination projetant un trait de lumière s'étendant transversalement à la direction de défilement. L'autre consiste, pour chaque rangée en cours d'examen, à procéder à phusieurs étapes de saisie d'images successives au cours du défilement, le nombre de telles étapes étant d'au moins deux, et de préférence de trois comme il a été décrit.

Il est en outre à noter que l'examen es oeuf est assuré sans le moindre contact mécanique avec la coquille des oeufs, grâce à l'utilisation de faisceaux finement focalisés, tels que produits par des sources laser.

## Revendications

1. Procédé de mirage des oeufs, consistant à examiner les œufs (1) par visiométrie dans des conditions d'illumination et de saisie d'images qui sont sensibles à un contraste lumineux s'observant au sein de chaque œuf, entre la zone liquide interne à la coquille de l'œuf et la zone d'air complémentaire de la chambre à air (6) existant éventuellement dans la coquille, dans lequel les œufs (1) défilent en rangées successives dans un poste de visiométrie comportant une caméra de saisie d'images numériques (3), **caractérisé en ce que** la caméra est sensible à la lumière rétrodiffusée à partir de l'intérieur de chaque œuf sous illumination par un faisceau de lumière focalisé sur l'œuf, sans contact mécanique, et où on soumet les signaux d'image obtenus à un traitement automatique suivant lequel on détermine, pour plusieurs points distincts en travers de chaque œuf, s'ils témoignent d'un niveau de contraste significatif de la présence d'une chambre à air par comparaison du niveau de contraste avec des valeurs prédéterminées.

2. Procédé de mirage des œufs suivant la revendication 1, **caractérisé en ce que** ledit contraste est observé sous illumination par un faisceau de lumière focalisé sur chaque œuf, sur une image de la lumière rétrodiffusée à partir de chaque œuf, à l'exclusion de la lumière renvoyée par réflexion sur sa coquille.

3. Procédé de mirage des œufs suivant la revendication 2, **caractérisé en ce que** ledit faisceau est sous forme d'un trait de lumière laser s'étendant en travers de chaque œuf examiné.

4. Procédé de mirage des œufs suivant l'une des revendications 1 à 3, **caractérisé en ce que**, par traitement des signaux d'image obtenus, on détermine la localisation de la chambre à air (6) dans chaque œuf.

5. Procédé de mirage des œufs suivant l'une quelconque des revendications précédentes, suivant lequel, pour chaque image saisie, les œufs de chaque rangée en cours d'examen sont soumis à illumination par un trait de lumière s'étendant transversalement à la direction de défilement.

6. Procédé de mirage des œufs suivant l'une quelconque des revendications précédentes, suivant lequel, pour chaque rangée d'œufs en cours d'examen, on procède à plusieurs étapes de saisie d'images successives au cours du défilement.

7. Procédé de mirage des œufs suivant l'une quelconque des revendications précédentes, **caractérisé en ce que** l'on dispose devant la caméra (3) un masque évitant son éblouissement par la lumière réfléchie par la coquille des oeufs.

8. Procédé suivant l'une quelconque des revendications précédentes, dans lequel les œufs sont illuminés individuellement par un faisceau laser, avantageusement en lumière infrarouge, et/ ou en ce que l'illumination et la saisie d'images s'effectue sensiblement suivant le même axe par rapport à chaque œuf, avantageusement par le dessus de rangées d'œufs examinés successivement.

9. Installation de mirage des œufs comprenant un poste de visiométrie à travers lequel les œufs (1) défilent par rangées successives, dans laquelle le poste de visiométrie comporte des moyens d'illumination individuelle des œufs par un faisceau de lumière focalisée et une caméra de saisie d'images numériques (3) sensible à la lumière rétrodiffusée à partir de chaque œuf, à l'exclusion de la lumière qui serait réfléchie par la coquille sans avoir pénétré à l'intérieur de l'œuf, ainsi que des moyens de traitement automatique des signaux d'images exploitant ces signaux pour déterminer, par calcul d'un niveau de contraste entre différentes zones de l'intérieur de l'œuf, respectivement sombre et claire, puis par comparaison dudit niveau de contraste avec des valeurs prédéterminées, si les images observées témoignent d'un contraste significatif de la présence d'une chambre à air (6) dans le champ de l'examen à l'intérieur de chaque œuf.

10. Installation suivant la revendication 9, dans laquelle moyens de traitement automatique des signaux d'images sont adaptés à déterminer la position de la chambre à air dans chaque œuf.

11. Installation suivant la revendication 9 ou 10, dans laquelle lesdits moyens de traitement automatique des signaux d'images sont en outre programmés pour fournir une indication de position du sommet de la chambre à air (6) dans chaque œuf.

12. Installation suivant la revendication 9, 10 ou 11, **caractérisée en ce que** lesdits moyens d'illumination sont adaptés à illuminer les œufs de chacune des rangées successives suivant un trait de lumière transversal à la direction de défilement suivant plusieurs étapes d'illumination successives pour chaque rangée au cours de leur défilement, et **en ce que** lesdits moyens de traitement d'images sont programmés pour opérer une sélection parmi les images (V1, V2, V3) saisies successivement pour chaque œuf en fonction du degré de contraste dont elles témoignent.

13. Procédé de suivi de l'incubation de lots d'œufs, **caractérisé en ce qu'**à un stade précoce de la croissance des embryons, on soumet ledit lot d'œufs aux étapes du procédé suivant l'une quelconque des revendications 1 à 8.

## Patentansprüche

1. Verfahren zur Durchleuchtung von Eiern, das darin besteht, die Eier (1) ohne mechanischen Kontakt durch Visiometrie in Beleuchtungs- und Bilderfassungsbedingungen zu untersuchen, die gegenüber einem Lichtkontrast empfindlich sind, der im Inneren von jedem Ei zwischen der flüssigen Zone innerhalb der Schale des Eis und der ergänzenden Luftzone der Luftzelle (6) beobachtet wird, die gegebenenfalls in der Schale vorhanden ist, wobei die Eier (1) in aufeinanderfolgenden Reihen in einem Visiometriestand vorbeilaufen, der eine Kamera (3) zur Erfassung digitaler Bilder umfasst, **dadurch gekennzeichnet, dass** die Kamera gegenüber dem Licht empfindlich ist, das ausgehend vom Inneren von jedem Ei unter der Beleuchtung von einem auf das Ei fokussierten Lichtstrahl rückgestreut wird, und wo die erhaltenen Bildsignale einer automatischen Verarbeitung unterzogen werden, gemäß der für mehrere getrennte Punkte quer durch jedes Ei durch Vergleich des Kontrastniveaus mit vorbestimmten Werten bestimmt wird, ob sie ein Kontrastniveau zeigen, das für das Vorhandensein einer Luftzelle kennzeichnend ist.

2. Verfahren zur Durchleuchtung von Eiern nach Anspruch 1, **dadurch gekennzeichnet, dass** der Kontrast unter Beleuchtung von einem auf jedes Ei fokussierten Lichtstrahl auf einem Bild des ausgehend von jedem Ei rückgestreuten Lichts unter Ausschluss des durch Reflexion auf seiner Schale reflektierten Lichts beobachtet wird.

3. Verfahren zur Durchleuchtung von Eiern nach Anspruch 2, **dadurch gekennzeichnet, dass** der Strahl die Form einer Laser-Lichtlinie aufweist, die sich durch jedes untersuchte Ei erstreckt.

4. Verfahren zur Durchleuchtung von Eiern nach Anspruch 1 bis 3, **dadurch gekennzeichnet, dass** durch Verarbeitung der erhaltenen Bildsignale der Ort der Luftzelle (6) in jedem Ei bestimmt wird.

5. Verfahren zur Durchleuchtung von Eiern nach einem der vorhergehenden Ansprüche, wobei für jedes erfasste Bild die Eier von jeder gegenwärtig untersuchten Reihe der Beleuchtung von einer Lichtlinie unterzogen werden, die sich quer zur Vorbeilaufrichtung erstreckt.

6. Verfahren zur Durchleuchtung von Eiern nach einem der vorhergehenden Ansprüche, wobei für jede Reihe von gegenwärtig untersuchten Eiern während des Vorbeilaufens mehrere aufeinanderfolgende Schritte zur Erfassung von Bildern durchgeführt werden.

7. Verfahren zur Durchleuchtung von Eiern nach einem der vorhergehenden Ansprüche, **dadurch gekennzeichnet, dass** vor der Kamera (3) eine Maske angeordnet wird, die ihre Blendung von dem von der Schale der Eier reflektierten Licht verhindert.

8. Verfahren nach einem der vorhergehenden Ansprüche, wobei die Eier einzeln von einem Laser-Strahl, vorzugsweise mit Infrarotlicht, beleuchtet werden, und/oder dadurch, dass die Beleuchtung und die Erfassung von Bildern im Wesentlichen entlang der gleichen Achse in Bezug auf jedes Ei, vorzugsweise von über Reihen von aufeinanderfolgend untersuchten Eiern, erfolgt.

9. Einrichtung zur Durchleuchtung von Eiern, die einen Visiometriestand umfasst, den die Eier (1) in aufeinanderfolgenden Reihen durchlaufen, wobei der Visiometriestand Mittel zur einzelnen Beleuchtung der Eier mit einem fokussierten Lichtstrahl und eine Kamera (3) zur Erfassung digitaler Bilder, die gegenüber dem Licht empfindlich ist, das ausgehend von jedem Ei rückgestreut wird, unter Ausschluss des Lichts, das gegebenenfalls von der Schale reflektiert wird, ohne in das Innere des Eis eingedrungen zu sein, sowie Mittel zur automatischen Verarbeitung der Bildsignale umfasst, die diese Signale nutzen, um durch Berechnung eines dunklen beziehungsweise hellen Kontrastniveaus zwischen unterschiedlichen Zonen im Inneren des Eis, dann durch Vergleich des Kontrastniveaus mit vorbestimmten Werten zu bestimmen, ob die beobachteten Bilder einen Kontrast zeigen, der für das Vorhandensein einer Luftzelle (6) im Feld der Untersuchung im Inneren von jedem Ei kennzeichnend ist.

10. Einrichtung nach Anspruch 9, wobei die Mittel zur automatischen Verarbeitung der Bildsignale angepasst sind, um die Position der Luftzelle in jedem Ei zu bestimmen.

11. Einrichtung nach Anspruch 9 oder 10, wobei die Mittel zur automatischen Verarbeitung der Bildsignale ferner programmiert sind, um eine Angabe der Position der Spitze der Luftzelle (6) in jedem Ei zu liefern.

12. Einrichtung nach Anspruch 9, 10 oder 11, **dadurch gekennzeichnet, dass** die Beleuchtungsmittel angepasst sind, um die Eier von jeder von den aufeinanderfolgenden Reihen gemäß einer Lichtlinie, die quer zur Vorbeilaufrichtung ist, gemäß mehreren aufeinanderfolgenden Beleuchtungsschritten für jede Reihe im Laufe ihres Vorbeilaufens zu beleuchten, und dadurch, dass die Bildverarbeitungsmittel programmiert sind, um eine Auswahl unter den aufeinanderfolgend für jedes Ei erfassten Bildern (V1, V2, V3) in Abhängigkeit vom Kontrastgrad, den sie zeigen, vorzunehmen.

13. Verfahren zur Verfolgung des Ausbrütens von Chargen von Eiern, **dadurch gekennzeichnet, dass** die Charge von Eiern in einem frühen Stadium des Wachstums der Embryonen den Schritten des Verfahrens nach einem der Ansprüche 1 bis 8 unterzogen wird.

## Claims

1. A method for candling eggs, consisting in examining the eggs (1) by a visiometric process under illumination and image capture conditions that are sensitive to a luminous contrast being observed within each egg, between the liquid area within the egg shell and the complementary air area of the air chamber (6) possibly existing in the shell, wherein the eggs (1) travel in successive rows within a visiometric station including a capture camera for digital images (3), **characterized in that** the camera is sensitive to the light retrodiffused from the interior of each egg under illumination by a light beam focused on egg, without mechanical contact, and where the obtained image signals are submitted to an automatic treatment wherein it is determined, for several distinct points across each egg, if they present a contrast level representative of the presence of an air chamber by comparison of the contrast level with predetermined values.

2. The method for candling eggs according to claim 1, **characterized in that** said contrast is observed under illumination by a light beam focused on each egg, on an image of the light retrodiffused from each egg, excluding the light returned by reflexion on its shell.

3. The method for candling eggs according to claim 2, **characterized in that** said beam is under the form of a streak of laser light extending across each examined egg.

4. The method for candling eggs according to one of claims 1 to 3, **characterized in that**, by treatment of the obtained image signals, the localization of the air chamber (6) in each egg is determined.

5. The method for candling eggs according to any of the preceding claims, wherein, for each captured image, the eggs of each row under examination are submitted to an illumination by a light streak extending transversally to the traveling direction.

6. The method for candling eggs according to any of the preceding claims, wherein, for each egg row under examination, several steps of successive image capture are carried out during traveling.

7. The method for candling eggs according to any of the preceding claims, **characterized in that** a mask avoiding camera dazzle by the light reflected by the egg shell is provided in front of the camera (3).

8. The method according to any of the preceding claims, wherein the eggs are illuminated individually by a laser beam, advantageously by infra-red light, and/or in that the illumination and the image capture are carried out substantially along the same axis relatively to each egg, advantageously by the top of successively examined egg rows.

9. An installation for candling eggs comprising a visiometric station through which the eggs (1) travel by successive rows, wherein the visiometric station includes means for individually illuminate the eggs by a focused light beam and a camera for capturing digital images (3) sensitive to the retrodiffused light from each egg, and excluding the light which would be reflected by the shell without having penetrated within the egg, as well as means for automatic treatment of the image signals using these signals to determine, by calculation of a contrast level between various areas, respectively dark and clear, within the egg, then by comparing said contrast level with predetermined values, whether or not the images observed present a contrast representative of the presence of an air chamber (6) in the examination field within each egg.

10. The installation according to claim 9, wherein the means for automatic treatment of the image signals are adapted to determine the position of the air chamber in each egg.

11. The installation according to claim 9 or 10, wherein said means for automatic treatment of the image signals are further programmed to provide an position indication of the top of the air chamber (6) into each egg.

12. The installation according to claim 9, 10 or 11, **characterized in that** said illumination means are adapted to illuminate the eggs of each of the successive rows according to a streak of light transversally to the traveling direction according to several successive illumination steps for each row during their travel, and **in that** said means for image processing are programmed to achieve a selection among the images (V1, V2, V3) successively captured for each egg according to the contrast degree that they provide.

13. A method for following-up the incubation of egg batches, **characterized in that**, at an early stage of the embryos growth, said egg batches are submitted to the steps of the method according to any of claims 1 to 8.
